# EUROPEAN PATENT APPLICATION

(11) **EP 3 906 850 A1**
(43) Date of publication of application: **10.11.2021**
(21) Application number: 20173611.3
(22) Date of filing: 08.05.2020
(51) Int. Cl.: A61B 5/055, A61B 5/00, G01R 33/28, G01R 33/385

(54) **APPARATUS FOR DETECTION OF PERIPHERAL NERVE STIMULATION**

(71) Applicant: Koninklijke Philips N.V., 5656 AG Eindhoven (NL)
(72) Inventor: WEISS, Steffen, 5656 AE Eindhoven (NL)
(74) Representative: Philips Intellectual Property & Standards

(57) **Abstract**

The present invention relates to an apparatus (10) for detection of peripheral nerve stimulation. The apparatus comprises an input unit (20); at least one sensor (30); a processing unit (40); and an output unit (50). The input unit is configured to provide the processing unit with information on individual scans of a scan sequence of a Magnetic Resonance Imaging "MRI" unit being used to perform medical imaging of a patient. The at least one sensor is configured to acquire sensor data of the patient undergoing the medical imaging. The processing unit is configured to determine a presence of peripheral nerve stimulation "PNS", wherein the determination of the presence of PNS comprises utilization of the information on the individual scans of the scan sequence and the sensor data of the patient. The output unit is configured to output an indication that the presence of PNS has been determined.

## Description

### FIELD OF THE INVENTION

The present invention relates to an apparatus for detection of peripheral nerve stimulation, an imaging system, a method for detection of peripheral nerve stimulation, as well as to a computer program element and a computer readable medium.

### BACKGROUND OF THE INVENTION

The strong currents applied to the gradient coils of a Magnetic Resonance imaging (MRI) unit during MRI procedures are known to have an undesirable side effect, which excites sensorial and motor nerves in the patient. The patient will feel this as a tickling sensation or spontaneous muscle contraction typically at the back or abdomen. This effect is known as peripheral nerve stimulation (PNS). It is uncomfortable and can even be painful for the patients and thus should be avoided during scanning. PNS may also cause motion artefacts, where the patient moves too much because of the PNS and this can compromise the imaging be undertaken. Patients can also become distressed, and in certain situations, may panic and make large movements that can be dangerous.

In a standard clinical setting staff help to minimize the possibility of PNS occurring by proper patient positioning, cable routing, and supervision during scanning. Expert staff can interpret a response of a patient as being indicative of PNS occurring. As a consequence staff can also adapt scans when they are causing PNS, to mitigate the PNS occurring. However sensitive patients may trigger a scan abort even due to mild PNS, which is not dangerous. When supervised, this can usually be handled by staff calming a patient down.

However, such staff intervention is not possible in an autonomous setting, and sedated, impaired, or non-knowledgeable patients, may not feel or report PNS, which may cause pain or motion artefacts.

There is a need to address these issues.

### SUMMARY OF THE INVENTION

It would be advantageous to have improved means of detecting peripheral nerve stimulation in a patient undergoing an MRI image scan. The object of the present invention is solved with the subject matter of the independent claims, wherein further embodiments are incorporated in the dependent claims. It should be noted that the following described aspects and examples of the invention apply also to the apparatus for detection of peripheral nerve stimulation, the imaging system, the method for detection of peripheral nerve stimulation, as well as to a computer program element and a computer readable medium.

In a first aspect, there is provided an apparatus for detection of peripheral nerve stimulation, the apparatus comprising:
- an input unit;
- at least one sensor;
- a processing unit; and
- an output unit.

The input unit is configured to provide the processing unit with information on individual scans of a scan sequence of a Magnetic Resonance Imaging "MRI" unit being used to perform medical imaging of a patient. The at least one sensor is configured to acquire sensor data of the patient undergoing the medical imaging. The processing unit is configured to determine a presence of peripheral nerve stimulation "PNS". The determination of the presence of PNS comprises utilization of the information on the individual scans of the scan sequence and the sensor data of the patient. The output unit is configured to output an indication that the presence of PNS has been determined.

The above developed apparatus is based on the insight of the inventors working in this field of technology, that peripheral nerve stimulation (PNS) when it occurs, occurs at a frequency defined by the repetition rate of the individual scans of a scan sequence. This is because, the gradient changes of coils used in each scan frequently repeat from one scan to the next over an imaging block, and it is the steep rising and falling slopes of very strong gradient globes for the coils that cause PNS. Thus, sensor data of the patient and information on the individual scans of a scan sequence can be utilised together to detect movements or artifacts relating to the patient that indicate the presence of PNS. This then enables, for example, staff to be alerted, the MR sequence to be adapted to avoid PNS, and indeed in severe cases the scan itself to be stopped.

In an example, the information on the individual scans of the scan sequence comprises a time period of the individual scans, and wherein the determination of the presence of PNS comprises utilization of the time period of the individual scans.

In an example, the processing unit is configured to select a section of sensor data over an acquisition time period. The determination of the presence of PNS can comprise utilization of the section of sensor data over the acquisition time period transformed into the frequency domain.

In an example, transformation into the frequency domain comprises utilization of a Fourier transform.

In an example, the processing unit is configured to select sensor data in the frequency domain at a centre frequency equal to the reciprocal of the time period of the individual scans. The determination of the presence of PNS can comprise utilization of the selected sensor data in the frequency domain at the centre frequency.

In an example, the processing unit is configured to determine a signal strength for the sensor data in the frequency domain at the centre frequency, and wherein the determination of the presence of PNS comprises utilization of the signal strength.

In this manner, the signal component of sensor data caused by PNS is selected, and the signal at all other frequencies is discarded enabling PNS to be detected with high signal to noise ratio.

In an example, the processing unit is configured to determine a signal strength for the sensor data in the frequency domain over a frequency range. The determination of the presence of PNS can comprise utilization of the signal strength for the sensor data in the frequency domain over the frequency range.

In other words, the signal strength associated with PNS, can be compared to a signal, such as a mean signal, over a frequency range, where the signal over the frequency range can be a good approximation of the noise floor.

In an example, the frequency range is centred at a frequency greater than the centre frequency equal to the reciprocal of the time period of the individual scans.

In this way, for example only the high frequency range of the spectrum can be selected for noise determination.

In an example, the information on the individual scans of the scan sequence comprises gradient waveform information for at least one gradient coil of the MRI unit. The determination of the presence of PNS can comprise utilization of the gradient waveform information for a gradient coil of the MRI unit.

In an example, the gradient waveform information for the gradient coil comprises at least one time of at least one gradient within a time period of the individual scans. The processing unit is configured to determine a correlation between a time of a gradient within the time period and an associated sensor data of the sensor data within the time period. The determination of the presence of PNS can comprise utilization of the correlation between the gradient and the associated sensor data.

In an example, the determination of the correlation between the gradient and the associated sensor data comprises a determination of a fixed phase relation between a time of the gradient in consecutive time periods of individual scans and a time of the associated sensor data in the consecutive time periods of individual scans.

Thus, even though in the temporal domain PNS induced movement of the patient will lag in time somewhat behind the steep gradients that cause the PNS, but by correlating the gradients in consecutive scans with sensor data, specific sensor data that occurs at a set phase, in other words a delay in time after the time of gradients, an indication can be made that the specific sensor data is indicative of PNS, and a determination can be made that PNS is occurring.

In a second aspect, there is provided an imaging system, comprising:
- a Magnetic Resonance Imaging "MRI" unit;
- at least one sensor; and
- a processing unit.

The MRI unit is configured to provide the processing unit with information on individual scans of a scan sequence of the MRI unit being used to perform medical imaging of a patient. The at least one sensor is configured to acquire sensor data of the patient undergoing the medical imaging. The processing unit is configured to determine a presence of peripheral nerve stimulation "PNS". The determination of the presence of PNS comprises utilization of the information on the individual scans of the scan sequence and the sensor data of the patient.

In an example, if a presence of PNS is determined, the processing unit is configured to control the MRI unit to adjust a gradient power of a coil of the MRI unit.

In a third aspect, there is provided a method for detection of peripheral nerve stimulation, the method comprising:
a) providing a processing unit with information on individual scans of a scan sequence of a Magnetic Resonance Imaging "MRI" unit being used to perform medical imaging of a patient;
b) acquiring by at least one sensor data of the patient undergoing the medical imaging;
c) determining by the processing unit a presence of peripheral nerve stimulation "PNS", wherein the determining the presence of PNS comprises utilizing the information on the individual scans of the scan sequence and the sensor data of the patient; and
d) optionally outputting an indication that the presence of PNS has been determined.

According to another aspect, there is provided a computer program element controlling one or more of the apparatuses or system as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic set up of an example of an apparatus for detection of peripheral nerve stimulation;
Fig. 2 shows a schematic set up of an example of an imaging system;
Fig. 3 shows a method for detection of peripheral nerve stimulation; and
Fig. 4 shows an exemplar sequence diagram of an MRI diffusion scan.

### DETAILED DESCRIPTION OF EMBODIMENTS

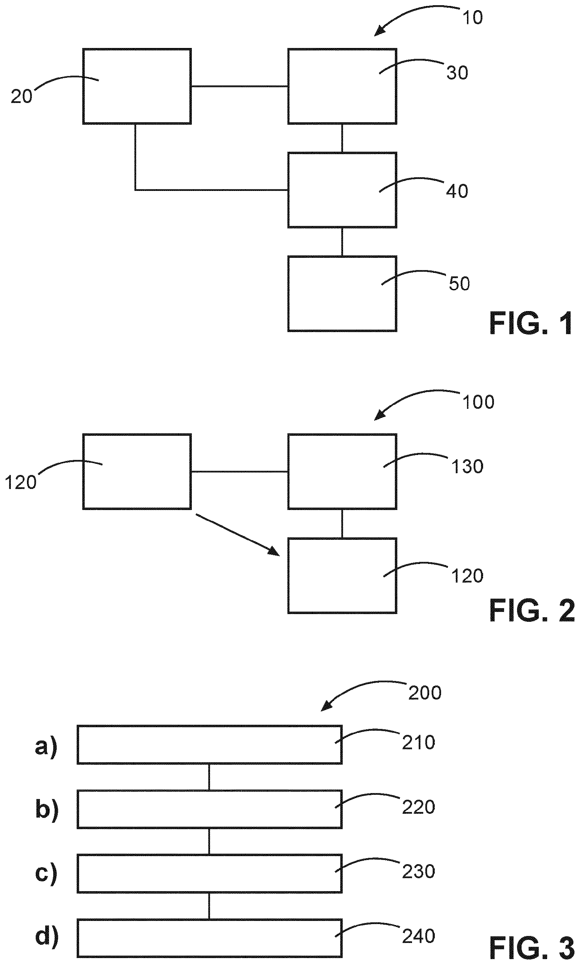

Fig. 1 shows a schematic example of an apparatus 10 for detection of peripheral nerve stimulation. The apparatus 10 comprises an input unit 20, at least one sensor 30, a processing unit 40, and an output unit 50. The input unit is configured to provide the processing unit with information on individual scans of a scan sequence of a Magnetic Resonance Imaging "MRI" unit being used to perform medical imaging of a patient. The at least one sensor is configured to acquire sensor data of the patient undergoing the medical imaging. The processing unit is configured to determine a presence of peripheral nerve stimulation "PNS". The determination of the presence of PNS by the processing unit comprises utilization of the information on the individual scans of the scan sequence and the sensor data of the patient. The output unit is configured to output an indication that the presence of PNS has been determined. The output unit could be part of the processing unit, and not be a separate unit as such.

According to an example, the information on the individual scans of the scan sequence comprises a time period of the individual scans. The determination of the presence of PNS by the processing unit can then comprise utilization of the time period of the individual scans.

According to an example, the processing unit is configured to select a section of sensor data over an acquisition time period. The determination of the presence of PNS by the processing unit can then comprise utilization of the section of sensor data over the acquisition time period transformed into the frequency domain.

According to an example, transformation into the frequency domain comprises utilization of a Fourier transform by the processing unit.

According to an example, the processing unit is configured to select sensor data in the frequency domain at a centre frequency equal to the reciprocal of the time period of the individual scans. The determination of the presence of PNS by the processing unit can then comprise utilization of the selected sensor data in the frequency domain at the centre frequency.

According to an example, the processing unit is configured to determine a signal strength for the sensor data in the frequency domain at the centre frequency. The determination of the presence of PNS by the processing unit can then comprise utilization of the signal strength.

According to an example, the processing unit is configured to determine a signal strength for the sensor data in the frequency domain over a frequency range. The determination of the presence of PNS by the processing unit can comprise utilization of the signal strength for the sensor data in the frequency domain over the frequency range.

According to an example, the frequency range is centred at a frequency greater than the centre frequency equal to the reciprocal of the time period of the individual scans.

In an example, the frequency range is centred at a frequency equal to the centre frequency equal to the reciprocal of the time period of the individual scans.

In an example, the signal strength for the sensor data in the frequency domain over the frequency range is determined as a mean signal strength for the sensor data in the frequency domain over the whole frequency range.

Thus, for example if the strength of the signal at the centre frequency is greater than the mean signal over all frequencies by a user-defined factor that is greater than 1, then the presence of PNS can be flagged. The user defined factor can be any value greater than 1, where the actual value can be determined based on the noise floor. Also, different values can be used to trigger different events, because as the PNS factor increases above 1, so the degree of PNS is increasing. Thus, a low level somewhat above 1 could be used to indicate to a staff member to reassure the patient, whilst a larger value of PNS further above 1 could be used to automatically adjust the scan or indicate to a staff member to adjust the scan, such that the new scan sequence is less likely to induce PNS in the patient. And a further increased value of PNS could be used to automatically stop the scan if necessary.

According to an example, the information on the individual scans of the scan sequence comprises gradient waveform information for at least one gradient coil of the MRI unit. The determination of the presence of PNS by the processing unit can then comprise utilization of the gradient waveform information for a gradient coil of the MRI unit.

According to an example, the gradient waveform information for the gradient coil comprises at least one time of at least one gradient within a time period of the individual scans. The processing unit is configured to determine a correlation between a time of a gradient within the time period and an associated sensor data of the sensor data within the time period. The determination of the presence of PNS by the processing unit can then comprise utilization of the correlation between the gradient and the associated sensor data.

According to an example, the determination of the correlation between the gradient and the associated sensor data by the processing unit comprises a determination of a fixed phase relation between a time of the gradient in consecutive time periods of individual scans and a time of the associated sensor data in the consecutive time periods of individual scans.

In an example, the at least one sensor comprises one or more of: one or more cameras; one or more position sensors; one or more acceleration sensors; one or more RF sensors; the MRI unit itself.

Fig. 2 shows a schematic example of an imaging system 100. The imaging system 100 comprises a Magnetic Resonance Imaging "MRI" unit 110, at least one sensor 120, and a processing unit 130. The MRI unit is configured to provide the processing unit with information on individual scans of a scan sequence of the MRI unit being used to perform medical imaging of a patient. The at least one sensor is configured to acquire sensor data of the patient undergoing the medical imaging. The processing unit is configured to determine a presence of peripheral nerve stimulation "PNS". The determination of the presence of PNS comprises utilization of the information on the individual scans of the scan sequence and the sensor data of the patient.

According to an example, if a presence of PNS is determined, the processing unit is configured to control the MRI unit to adjust a gradient power of a coil of the MRI unit.

In an example, the information on the individual scans of the scan sequence comprises a time period of the individual scans. The determination of the presence of PNS by the processing unit can then comprise utilization of the time period of the individual scans.

In an example, the processing unit is configured to select a section of sensor data over an acquisition time period. The determination of the presence of PNS by the processing unit can then comprise utilization of the section of sensor data over the acquisition time period transformed into the frequency domain.

In an example, transformation into the frequency domain comprises utilization of a Fourier transform by the processing unit.

In an example, the processing unit is configured to select sensor data in the frequency domain at a centre frequency equal to the reciprocal of the time period of the individual scans. The determination of the presence of PNS by the processing unit can then comprise utilization of the selected sensor data in the frequency domain at the centre frequency.

In an example, the processing unit is configured to determine a signal strength for the sensor data in the frequency domain at the centre frequency. The determination of the presence of PNS by the processing unit can then comprise utilization of the signal strength.

In an example, the processing unit is configured to determine a signal strength for the sensor data in the frequency domain over a frequency range. The determination of the presence of PNS by the processing unit can then comprise utilization of the signal strength for the sensor data in the frequency domain over the frequency range.

In an example, the frequency range is centred at a frequency greater than the centre frequency equal to the reciprocal of the time period of the individual scans

In an example, the signal strength for the sensor data in the frequency domain over the frequency range is determined as a mean signal strength for the sensor data in the frequency domain over the whole frequency range.

In an example, the information on the individual scans of the scan sequence comprises gradient waveform information for at least one gradient coil of the MRI unit. The determination of the presence of PNS by the processing unit can then comprise utilization of the gradient waveform information for a gradient coil of the MRI unit.

In an example, the gradient waveform information for the gradient coil comprises at least one time of at least one gradient within a time period of the individual scans. The processing unit is configured to determine a correlation between a time of a gradient within the time period and an associated sensor data of the sensor data within the time period. The determination of the presence of PNS by the processing unit can then comprise utilization of the correlation between the gradient and the associated sensor data.

In an example, the determination of the correlation between the gradient and the associated sensor data by the processing unit comprises a determination of a fixed phase relation between a time of the gradient in consecutive time periods of individual scans and a time of the associated sensor data in the consecutive time periods of individual scans.

In an example, the at least one sensor comprises one or more of: one or more cameras; one or more position sensors; one or more acceleration sensors; one or more RF sensors.

In an example, the MRI unit is configured to MRI data useable as the sensor data useable to determine PNS.

In an example, the system is configured to output an indication that the presence of PNS has been determined.

Fig. 3 shows an example of a method 20) for detection of peripheral nerve stimulation in its basic steps. The method comprises:
- in a providing step 210, also referred to as a), providing a processing unit with information on individual scans of a scan sequence of a Magnetic Resonance Imaging "MRI" unit being used to perform medical imaging of a patient;
- in an acquiring step 220, also referred to as step b), acquiring by at least one sensor sensor data of the patient undergoing the medical imaging; and
- in a determining step 230, also referred to as step c), determining by the processing unit a presence of peripheral nerve stimulation "PNS", wherein the determining the presence of PNS comprises utilizing the information on the individual scans of the scan sequence and the sensor data of the patient.

In an example, the method comprises an outputting step 240, also referred to as step d), where an indication that the presence of PNS has been determined is output.

In an example, if a presence of PNS is determined, the method comprises controlling by the processing unit the MRI unit to adjust a gradient power of a gradient coil of the MRI unit.

In an example, the information on the individual scans of the scan sequence comprises a time period of the individual scans, and wherein step c) comprises utilizing the time period of the individual scans.

In an example, the method comprises selecting by the processing unit a section of sensor data over an acquisition time period, and wherein step c) comprises utilizing the section of sensor data over the acquisition time period transformed into the frequency domain.

In an example, transformation into the frequency domain comprises utilizing a Fourier transform.

In an example, the method comprises selecting by the processing unit sensor data in the frequency domain at a centre frequency equal to the reciprocal of the time period of the individual scans, and wherein step c) comprises utilizing the selected sensor data in the frequency domain at the centre frequency.

In an example, the method comprises determining by the processing unit a signal strength for the sensor data in the frequency domain at the centre frequency, and wherein step c) comprises utilizing the signal strength.

In an example, the method comprises determining by the processing unit a signal strength for the sensor data in the frequency domain over a frequency range, and wherein step c) comprises utilizing the signal strength for the sensor data in the frequency domain over the frequency range.

In an example, the frequency range is centred at a frequency greater than the centre frequency equal to the reciprocal of the time period of the individual scans.

In an example, the signal strength for the sensor data in the frequency domain over the frequency range is determined as a mean signal strength for the sensor data in the frequency domain over the whole frequency range.

In an example, the information on the individual scans of the scan sequence comprises gradient waveform information for at least one gradient coil of the MRI unit, and wherein step c) comprises utilizing the gradient waveform information for a gradient coil of the MRI unit.

In an example, the gradient waveform information for the gradient coil comprises at least one time of at least one gradient within a time period of the individual scans, wherein the method comprises determining by the processing unit a correlation between a time of a gradient within the time period and an associated sensor data of the sensor data within the time period, and wherein step c) comprises utilizing the correlation between the gradient and the associated sensor data.

In an example, the determining the correlation between the gradient and the associated sensor data comprises determining a fixed phase relation between a time of the gradient in consecutive time periods of individual scans and a time of the associated sensor data in the consecutive time periods of individual scans.

In an example, the at least one sensor comprises one or more of: one or more cameras; one or more position sensors; one or more acceleration sensors; one or more RF sensors; the MRI unit itself.

Thus, a new technique of automatically detecting PNS is based on the insight that PNS occurs at a frequency defined by the repetitive gradient waveform of the MRI scan, because the root cause of PNS is the induction of voltages in the body caused by fast changes of the magnetic field generated by the gradient coils. This insight is exploited to realize a highly sensitive PNS sensing apparatus/system by spectrally-selective detection of PNS from various types of sensors, such as cameras, or position sensors, or acceleration sensors, or even using image data from the MRI unit itself.

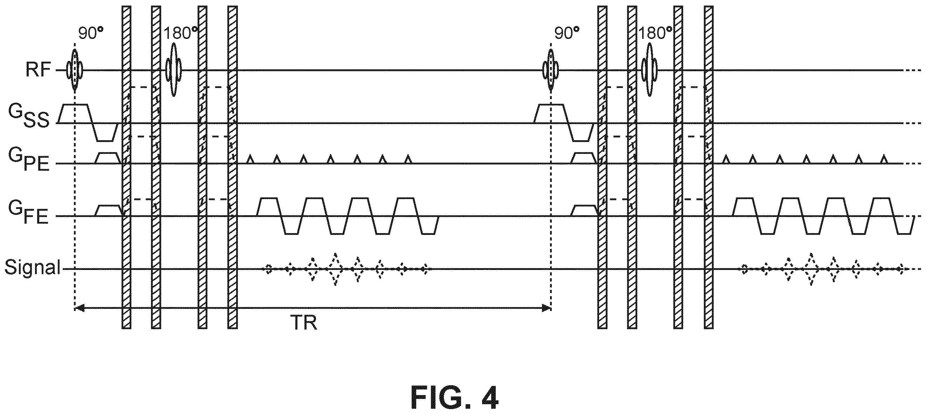

The apparatus for detection of peripheral nerve stimulation, the imaging system, and the method for detection of peripheral nerve stimulation are now described in further detail with respect to specific embodiments, where reference is made to Fig. 4.

Fig. 4 shows a typical MRI sequence that commonly causes PNS. Fig. 4 is a representation of a sequence diagram of MRI diffusion scan. The lines Gss, G_{PE}, and G_{FE} depict the gradient waveforms. The diffusion gradient lobes shown with dotted lines between the vertical bars have very steep rising and falling slopes and the magnitudes are actually much higher than drawn here. Such gradients can cause PNS due to the steep rising and falling slopes. Individual scans of a scan block are repeated, at a repetition time of TR.

Continuing with Fig. 4, this diffusion sequence therefore comprises two very strong gradient lobes, with steep rising and falling slopes at four time points in the repetition time of each scan of a scan block or scan sequence. Their steep rising and falling slopes cause PNS and are marked with the vertical bars. As discussed, the basic building block of the sequence lasts one repetition time TR, which can for example be 800ms. This block is repeated many times during sequence execution so that the gradients are repeated at a respective frequency of 1.25Hz. It has been found by the inventors that PNS is caused in synchrony with this repetition, i.e. at the same basic frequency, and even in a fixed phase relation to the waveform due for example to movement of the patient slightly lagging in time to the steep gradients. It is to be noted that other examples of scans that cause PNS can have shorter TRs and higher repetition frequencies, and can also have longer TRs.

As the patient undergoes an MRI scan, sensor data of the patient is acquired, and is analyzed along with the information of the individual scans, such as that shown in Fig. 4. Then a sensitive quantitative measure of PNS is generated by a spectrally-selective evaluation of the output of any sensor. This can be achieved for example by a Fourier-based analysis in the frequency domain as explained below, but can in principle also be done by a gating mechanism in the time-domain, and also can be done in the time domain by a correlation of the slopes of the gradients with the sensor data within and between consecutive scans to determine a constant phase relation between the times of specific sensor data and the times of the slopes.

The following relates to specific examples of how this can be achieved.

### Frequency domain

The output of the time signal of a sensor is sampled. Sections with a length of the time period T_{acq} are then Fourier-transformed, and the mean strength P of the signal in the frequency band Δf=1/TR +/- 1/T_{acq} is measured, whereas signal at all other frequencies is discarded. This effectively selects the signal component caused by PNS. The acceptation bandwidth 2/T_{acq} is chosen because this defines the principal limit of frequency resolution of any time signal that is sampled for a time T_{acq}. This is governed by the Nyquist limit.

The Fourier analysis can be done in a sliding window mode, i.e., after the Fourier transform of the first fully sampled data vector of duration T_{acq} is acquired it is not required to wait for T_{acq} to fill the next data vector entirely with new data, but the next Fourier transform can already be done after replacing, for example, only a quarter of the data. This could of course occur at the half-data point or the 10% point for example.

The presence of PNS can be determined in the following manner. The strength P of the signal in the frequency band Δf=1/TR +/- 1/T_{acq} is compared to the mean signal M over all frequencies, which for a patient who lies still is a good approximation of the noise floor. Rather than use all the frequencies here for the noise floor, a portion of all the frequencies can for example be used, which could for example be a high frequency band above the frequency band Δf. If the P > f^{∗}M with a user-defined factor f>1, then the presence of PNS is flagged. This flag may automatically trigger actions such as:
alerting staff
change of the sequence into a mode that uses less gradient power (in mT/m) and / or speed (in mT/m/ms)
stopping the scan
Different factors fᵢ can be defined for different actions. For example, a low factor may be used for alerting staff, while a high factor may be used to stop the scan immediately due to a high level of PNS having been detected.

### Time-domain

Principally, a gating mechanism is applied to read out any sensor only during the phases in which PNS occurs. It can be known what temporal delay PNS has to a gradient slope, or calibration can be carried out, and the sensor data can be gated over a short time period delayed from the times of the gradient slopes. The magnitude of the sensor data in this gated window can then be used to determine PNS, and can be compared in a similar manner to that described above for the frequency domain to an average signal over the whole period TR (or a required portion of TR) to determine if PNS has occurred, again using an indicator that the signal over the gated time window be some user defined magnitude greater than the magnitude over the whole period TR (or other portion of TR) as an indicator of PNS.

However, an algorithm can be used to correlate the sensor data and the times of specific sensor within each period TR with the magnitudes and times of the gradient slopes to establish if specific sensor data shows a fixed phase relation to gradient slopes in consecutive time periods TR. In other words, the algorithm establishes if an elevated sensor signal appears at a fixed time after the time of a gradient slope, and this can be used to indicate that the sensor data is associated with PNS and it is automatically indicated that PNS has been detected.

### Cameras as motion sensors

Cameras can be used to monitor the patient. These can be used to monitor those regions of the patient where it is know that PNS occurs. Because PNS motion is often subtle, patient clothing and coils can be designed with patterns providing fine detail rather than large uniform-coloured surfaces. A high frame rate can be used for the camera, which can be beneficial for scans with a short TR. By setting the camera frame rate at a multiple of 1/TR, after Fourier transform, a benefit is that all signal due to PNS falls exactly into one bin of the output vector. However, this frame rate is not essential.

### Location (position) / acceleration sensors as motion sensors

Known location (position) or acceleration sensors can be used as motion sensors. The signal of acceleration sensors can be integrated once or twice over time to actually use the velocity or position information of the patient. Acceleration sensors have the benefit that they are based on micro-electro-mechanical systems and semiconductors, and can be made MR compatible and very compact. Position sensors based on fiber-optic principles can be used because they are MR safe.

One or multiple location or acceleration sensors can therefore be fixed to the patient or into clothing worn by the patient or into the MR coils. Again, these can be located at regions of the patient that are likely to be subjected to PNS, such as the upper and lower back and stomach.

### MRI signal as sensor

Many MRI sequences acquire image data in a way that allows to reconstruct lowly resolved MR images often with a reduced number of dimensions. An example is a sequence that frequently acquires one-dimensional projections of the field of view. This dynamic image data can then be used as a position sensor.

### Non-contact sensors

Non-contact sensors based on RF-measurements of the patient can also be used as position sensors.

### Adaptation of the scan sequence

If PNS is detected, specific predefined sequence adaptations can performed "on the fly" during scanning to automatically lower the gradient power to avoid PNS. Thus, the strength of the gradient lobes with the steepest slopes and highest strengths can lowered in several small steps until the PNS signal vanishes. This mechanism lowers the gradient power only to the extent required such that PNS is just avoided and keeps the scan as fast as possible, and takes the individual PNS threshold of the patient into account. This can be very beneficial, because PNS thresholds for patients are known to vary widely.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for detection of peripheral nerve stimulation, the apparatus comprising:
- an input unit (20);
- at least one sensor (30);
- a processing unit (40); and
- an output unit (50);
wherein, the input unit is configured to provide the processing unit with information on individual scans of a scan sequence of a Magnetic Resonance Imaging "MRI" unit being used to perform medical imaging of a patient;
wherein, the at least one sensor is configured to acquire sensor data of the patient undergoing the medical imaging;
wherein, the processing unit is configured to determine a presence of peripheral nerve stimulation "PNS", wherein the determination of the presence of PNS comprises utilization of the information on the individual scans of the scan sequence and the sensor data of the patient; and
wherein, the output unit is configured to output an indication that the presence of PNS has been determined.

2. Apparatus according to claim 1, wherein the information on the individual scans of the scan sequence comprises a time period of the individual scans, and wherein the determination of the presence of PNS comprises utilization of the time period of the individual scans.

3. Apparatus according to claim 2, wherein the processing unit is configured to select a section of sensor data over an acquisition time period, and wherein the determination of the presence of PNS comprises utilization of the section of sensor data over the acquisition time period transformed into the frequency domain.

4. Apparatus according to claim 3, wherein transformation into the frequency domain comprises utilization of a Fourier transform.

5. Apparatus according to any of claims 3-4, wherein the processing unit is configured to select sensor data in the frequency domain at a centre frequency equal to the reciprocal of the time period of the individual scans, and wherein the determination of the presence of PNS comprises utilization of the selected sensor data in the frequency domain at the centre frequency.

6. Apparatus according to claim 5, wherein the processing unit is configured to determine a signal strength for the sensor data in the frequency domain at the centre frequency, and wherein the determination of the presence of PNS comprises utilization of the signal strength.

7. Apparatus according to claim 6, wherein the processing unit is configured to determine a signal strength for the sensor data in the frequency domain over a frequency range, and wherein the determination of the presence of PNS comprises utilization of the signal strength for the sensor data in the frequency domain over the frequency range.

8. Apparatus according to claim 7, wherein the frequency range is centred at a frequency greater than the centre frequency equal to the reciprocal of the time period of the individual scans.

9. Apparatus according to any of claims 1-8, wherein the information on the individual scans of the scan sequence comprises gradient waveform information for at least one gradient coil of the MRI unit, and wherein the determination of the presence of PNS comprises utilization of the gradient waveform information for a gradient coil of the MRI unit.

10. Apparatus according to claim 9, wherein the gradient waveform information for the gradient coil comprises at least one time of at least one gradient within a time period of the individual scans, wherein the processing unit is configured to determine a correlation between a time of a gradient within the time period and an associated sensor data of the sensor data within the time period, and wherein the determination of the presence of PNS comprises utilization of the correlation between the gradient and the associated sensor data.

11. Apparatus according to claim 10, wherein the determination of the correlation between the gradient and the associated sensor data comprises a determination of a fixed phase relation between a time of the gradient in consecutive time periods of individual scans and a time of the associated sensor data in the consecutive time periods of individual scans.

12. An imaging system (100), comprising:
- a Magnetic Resonance Imaging "MRI" unit (110);
- at least one sensor (120); and
- a processing unit (130);
wherein, the MRI unit is configured to provide the processing unit with information on individual scans of a scan sequence of the MRI unit being used to perform medical imaging of a patient;
wherein, the at least one sensor is configured to acquire sensor data of the patient undergoing the medical imaging; and
wherein, the processing unit is configured to determine a presence of peripheral nerve stimulation "PNS", wherein the determination of the presence of PNS comprises utilization of the information on the individual scans of the scan sequence and the sensor data of the patient.

13. System according to claim 12, wherein if a presence of PNS is determined, the processing unit is configured to control the MRI unit to adjust a gradient power of a coil of the MRI unit.

14. A method (200) for detection of peripheral nerve stimulation, the method comprising:
a) providing (210) a processing unit with information on individual scans of a scan sequence of a Magnetic Resonance Imaging "MRI" unit being used to perform medical imaging of a patient;
b) acquiring (220) by at least one sensor sensor data of the patient undergoing the medical imaging;
c) determining (230) by the processing unit a presence of peripheral nerve stimulation "PNS", wherein the determining the presence of PNS comprises utilizing the information on the individual scans of the scan sequence and the sensor data of the patient; and
d) optionally outputting (240) an indication that the presence of PNS has been determined.

15. A computer program element for controlling an apparatus according to one of claims 1 to 11 and/or system of any of claims claim 12, which when executed by a processor is configured to carry out the method of claim 14.
